(19) Europäisches Patentamt
European Patent Office
Office européen des brevets

(11) Veröffentlichungsnummer: **0 149 093**
**B1**

# (12) EUROPÄISCHE PATENTSCHRIFT

(45) Veröffentlichungstag der Patentschrift: **31.05.89**

(51) Int. Cl.⁴: **C 07 H 13/12**, C 07 H 15/00, A 61 K 31/70

(21) Anmeldenummer: **84114659.0**

(22) Anmeldetag: **03.12.84**

(54) **N-glycosylierte Harnstoffe, Carbamate und Thiocarbamate, Verfahren zu ihrer Herstellung sowie ihre Verwendung.**

(30) Priorität: **14.12.83 DE 3345250**
**23.12.83 DE 3346623**

(43) Veröffentlichungstag der Anmeldung:
**24.07.85 Patentblatt 85/30**

(45) Bekanntmachung des Hinweises auf die Patenterteilung:
**31.05.89 Patentblatt 89/22**

(84) Benannte Vertragsstaaten:
**AT BE CH DE FR GB IT LI NL SE**

(56) Entgegenhaltungen:
**EP-A-0 091 645**

**CHEMICAL ABSTRACTS, Band 59, Nr. 13, 23. Dezember 1963, Spalte Nr. 15370c-f, Columbus, Ohio, US; A, MASUO et al.: "Structure of ro-ethoxyphenylurea-N-glucuronide", & CHEM. PHARM. BULL. (Tokyo) 11(9),1214-1(1963)"**
**CARBOHYDRATE RESEARCH, vol. 88, no. 1, Januar 1981, Seiten 61-75, Elsevier Scientific Publishing Co., Amsterdam, NL; H. TSUTSUMI et al.: "Synthesis of phenyl D-glucopyranosides; nucleophilic substitution of O-(2,3,4,6-tetra-O-benzyl-D-glucopyranosyl)-pseudoureas by phenols"**
**CHEMISCHE BERICHTE, Band 97, Nr. 11, November 1964, Seiten 3256-3261, Weinheim, DE; H. DORN et al.: "Verknüpfung mitosehemmender alpha-Pheyl-**

(73) Patentinhaber: **BAYER AG, D-5090 Leverkusen 1 Bayerwerk (DE)**

(72) Erfinder: **Lockhoff, Oswald, Dr., Morgengraben 14, D-5000 Köln 80 (DE)**
Erfinder: **Krüger, Bernd- Wieland, Dr., Sillerstrasse 49, D-5600 Wuppertal 11 (DE)**
Erfinder: **Stadler, Peter, Dr., Am Ideck 8, D-5657 Haan 1 (DE)**
Erfinder: **Metzger, Karl Georg, Dr., Pahlkestrasse 75, D-5600 Wuppertal 1 (DE)**
Erfinder: **Opitz, Hans- Georg, Dr., Steinberger Weg 52, D-5600 Wuppertal 11 (DE)**
Erfinder: **Schaller, Klaus, Dr., Am Sonnenschein 38, D-5600 Wuppertal 1 (DE)**
Erfinder: **Stünkel, Klaus Georg, Dr., Am Eckbusch 55, D-5600 Wuppertal 1 (DE)**
Erfinder: **Zeiler, Hans- Joachim, Dr, Elsbeekerstrasse 46, D-5620 Velbert 15 (DE)**

(56) Entgegenhaltungen: (Fortsetzung)
**beta- 4-alkoxy-phenyl -äthylamine mit D-Glucose"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 149 093 B1

LIBER, STOCKHOLM 1989

## Beschreibung

Die vorliegende Erfindung betrifft neue N-glycosylierte Harnstoffe, Carbamate und Thiocarbamate, Verfahren zu Ihrer Herstellung sowie ihre Verwendung zur Beeinflussung der körpereigenen Abwehr.

Zum Stand der Technik ist auszuführen, daß in Chemical Abstracts, 59: 15370 c - f einfach N-substituierte N-Glucuronide beschrieben werden. Die Arbeit von Dorn et al. in Chemische Bericht, Band 97, (1967), S. 3256 - 3261 beschreibt die gleichen Verbindungen und deren Eignung als Cytostatica.

Die Arbeit von Tsutsumi et al. in Carbohydrate Research, 88 (1981), S. 61 - 75 beschreibt N-Phenyl substituierte N-Glucopyranoside und Verfahren zu deren Herstellung. EP-A-91 645 beschreibt N-glycosylierte Carbonsäureamid-Derivate zur Beeinflussung der körpereigenen Abwehr.

Die neuen Verbindungen entsprechen der allgemeinen Formel I

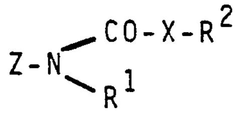

(I)

in der

- $R^1$ einen ggf. substituierten, cyclischen, geradkettigen oder verzweigten gesättigten oder ein- oder mehrfach ungesättigten Kohlenwasserstoffrest mit bis zu 21 C-Atomen darstellt,
- $R^2$ einen ggf. substituierten, aromatischen Kohlenwasserstoffrest oder einen Aralkylrest oder einen ggf. substituierten, cyclischen, geradkettigen oder verzweigten gesättigten oder ein- oder mehrfach ungesättigten Kohlenwasserstoffrest mit 10 - 25 C-Atomen darstellt,
- X für O, S, NH oder NR steht, worin R einen Alkylrest mit bis zu 20, vorzugsweise bis zu 10 C-Atomen darstellt und
- Z einen über das anomere Kohlenstoffatom gebundenen Glycosylrest darstellt,

$R^1$ in der Bedeutung Kohlenwasserstoffrest steht im allgemeinen für einen Alkyl-, Alkenyl-, Cycloalkyl- oder Cycloalkenylrest, wobei diese auch gemeinsam auftreten können, z. B. als Alkylcycloalkyl.

Im Rest $R^1$ in der Bedeutung Alkyl oder Alkenyl konnen auch einzelne, im allgemeinen bis zu 5, vorzugsweise 1 oder 2 Methylen- oder Methingruppen durch O, S oder N unterbrochen sein. Bei dieser unterbrechung der Kette durch N trägt dieses Stickstoffatom entweder H oder einen Alkylrest mit bis zu 20 Kohlenstoffatomen oder einen CO-Alkylrest, wobei diese Alkylgruppe bis zu 20 C-Atome vorzugsweise jeweils bis zu 10 C-Atomen aufweist.

Vorzugsweise besteht $R^1$ aus einem Kohlenwasserstoffrest mit 7 - 21 C-Atomen.

Ganz besonders bevorzugt steht $R^1$ für einen Alkyl- oder Alkenylrest mit 7 - 21 C-Atomen.

Beispielhaft für gesättigte Reste $R^1$ seien genannt:

Methyl, Ethyl, n-Propyl, i-Propyl, n-Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Eicosyl, Docosyl, Tetracosyl, Triacontyl, Ethylpentyl, Methyldecyl, i-Propyldecyl, Methyltridecyl, Pentylhexadecyl, 1-Dodecylhexadecyl, 2-Dodecylhexadecyl, 3-Dodecylhexadecyl, 1-Hexadecyloctadecyl, 2-Hexadecyloctadecyl, 3-Hexadecyloctadecyl, 4-Hexadecyloctadecyl, 1-Octadecyleicosyl, 2-Octadecyleicosyl.

Ungesättigte Reste sind beispielsweise:

Ethenyl, Propenyl-1, Propenyl-2, i-Butenyl, Butenyl-1, Butenyl-2, 1-Pentenyl, 2-Pentenyl, 3-Pentenyl, 4-Pentenyl 1-Hexenyl, 2-Hexenyl, 3-Hexenyl, 4-Hexenyl, 5-Hexenyl, 1-Decenyl, 5-Decanyl, 9-Decenyl, 2-Heptadecenyl, 1,3 Butadienyl, 1,3-Pentadienyl, 1,4-Pentadienyl, 8,11-Heptadecandienyl, 8,11,14-Heptadecantrienyl.

Im allgemeinen sind die längerkettigen, ungesättigten Reste bevorzugt, speziell die einfach oder zweifach ungesättigten Alkenyle mit 7 bis 21 Kohlenstoffatomen.

Die ungesättigten Kohlenwasserstoffreste können dabei als reine cis- oder trans-Isomere oder auch als Isomerengemische vorliegen.

Beispiele für cyclische Reste sind:

Cyclopropyl, Cyclobutyl, Cyclopentyl, Cyclohexyl, Cycloheptyl, Adamantyl, Cyclobutylcyclopentyl, Cyclopentylcyclopentyl, Cyclohexylcyclopentyl, Cyclohexylcyclohexyl, Cyclohexyladamantyl, Adamantylcyclohexyl, Dicyclohexylcyclopentyl, Decahydronaphthyl.

Beispielhaft für Alkylcycloalkylreste seien genannt:

Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexylhexyl, Cyclohexyldecyl, Cyclopentylcyclohexylethyl, Cyclohexylcyclopentylethyl und Cyclohexylcyclohexylethyl.

In den genannten Beispielen, in denen die Cycloalkylreste zweifach substituiert sind, können die Substituenten sowohl cis als auch trans zueinander angeordnet sein.

Beispiele in denen die Reste $R^1$ in Formel (I) durch Sauerstoff-, Schwefel- und/oder Stickstoffatome unterbrochene Kohlenwasserstoffreste darstellen, sind Methoxyethyl, Methoxyethoxyethyl, Dimethylaminoethyloxyethyl, Dibutylaminohexyl, N-Methylaminodecyl.

Die Reste $R^1$ können substituiert sein, vorzugsweise 1 bis 3-fach. Bevorzugte Substituenten hierfür sind $C_6$-, $C_{10}$- oder $C_{12}$-Aryl, Halogen, vorzugsweise Cl und Br, Amino, $C_1$-$C_6$-Alkylamino, Di-$C_1$-$C_6$-alkylamino, Oxo, OH, $C_1$-$C_6$-Alkoxy, SH, $C_1$-$C_6$-Alkylmercapto, $C_1$-$C_{20}$-Alkyl-COO und $C_1$-$C_{20}$-Alkyl-OC-NH.

Beispiele für derart substituierte Reste $R^1$ sind Mercaptoethyl, β-Hydroxytridecyl, ω-Hydroxyheptadecenyl, Oxobutyl, Aminodecyl, Chlorethyl, Fluormethyl, ω-Chlordodecyl, ω-Fluorhexadecyl, ω-Fluoroctadecyl, ω-Chlortetradecyl, ω-Chlorhexadecyl, ω-Chlorheptadecyl, ω-Chloroctadecyl, 2-Aminododecyl, 2-Aminohexadecyl, 2-Aminooctadecyl, 2-(Methylamino)-tetradecyl, 2-(Hexylamino)-octadecyl, 2-(Dimethylamino)-hexadecyl, 2-Oxotetradecyl, 4-Oxotetradecyl, 2-Oxooctadecyl, 2-Hydroxytetradecyl, 2-Hydroxyoctadecyl, 2-Methoxyoctadecyl, 2-(Hexyloxy)-tetradecyl, 4-Mercaptooctadecyl, 2-Methylthiotetradecyl, 3-Methylthiotetradecyl, 2-(Acetyloxy)-tetradecyl, 3-Methylthiotetradecyl, 2-(Acetyloxy)-tetradecyl, 2-(Lauroyloxy)-tetradecyl, 2-(Myristoyloxy)-hexadecyl, 2-(Acetylamido)-tetradecyl, 2-(Myristoylamido)-tetradecyl, ω-(Acetylamido)-tetradecyl.

Der Kohlenwasserstoffrest $R^2$ kann, ebenso wie der Rest $R^1$, ein Alkyl-, Alkenyl-, Cycloalkyl-, Cycloalkenyl- oder Arylrest sein, wobei ebenfalls diese Bedeutungen miteinander kombiniert vorliegen können wie für $R^1$ schon angegeben. In der gleichen Weise, wie für $R^1$ genannt, kann der Rest $R^2$ durch O, S oder NR unterbrochen sein.

Ferner kann $R^2$ nach Art und
zahl wie für $R^1$ angegeben substituiert sein.

Beispiele für Reste $R^2$ sind:

n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Eicosyl, Docosyl, Tetracosyl, Methyldecyl, i-Propyldecyl, Methyltridecyl, Pentylhexadecyl, 1-Decenyl, 5-Decenyl, 9-Decenyl, 2-Heptadecenyl, 1,3-Pentadienyl, 1,4-Pentadienyl, 8,11-Heptadecandienyl, 8,11,14-Heptadecantrienyl, Adamantyl, Cyclopentylcyclopentyl, Cyclohexylcyclopentyl, Cyclohexylcyclohexyl, Cyclohexyladamantyl, Adamantylcyclohexyl, Dicyclohexylcyclopentyl, Decahydronaphthyl, Octylcyclopentyl, Ethylcyclohexyl, Propylcyclohexyl, Butylcyclohexyl, Hexylcyclohexyl, Decylcyclohexyl, Cyclopentylpentyl, Cyclopentylhexyl, Cyclopentyloctyl, Cyclopentyldecyl, Cyclohexylethyl, Cyclohexylpropyl, Cyclohexylbutyl, Cyclohexylhexyl, Cyclohexyldecyl, Cyclopentylcyclohexylethyl, Cyclohexylcyclopentylethyl und Cyclohexylcyclohexylethyl, Biphenyl, Naphthyl, Hydroxynaphthyl, Phenylhexyl, Phenyldodecyl, p-Methoxyphenylhexyl, Naphthylbutyl, Dimethylaminoethyloxyethyl, Dibutylaminohexyl, β-Hydroxytridecyl, Hydroxyheptadecenyl, Aminodecyl, N-Methylaminodecyl, N-Octylaminododecyl, N-Dihexylaminotetradecyl, β-Acetyloxytridecyl, β-Lauroyloxytridecyl, β-Myristoyloxytridecyl, β-Palmitoyloxytridecyl, β-Stearoyloxytridecyl, β-Myristoyloxypentadecyl, β-Myristoyloxyheptadecyl, Stearoylamidopentadecyl, Acetylamidoheptadecyl, 2-(Methylthio)-tridecyl, 5-(Propylthio)-pentadecyl.

Z in Formel (I) bedeutet einen Glycosylrest, der bei den erfindungsgemäßen Formeln immer über das anomere Kohlenstoffatom mit der Urethan-, Thiocarbamat- bzw. Harnstoffgruppierung verbunden ist. Unter Glycosylrest sind erfindungsgemäß Mono-, Di- und Oligosaccharidreste zu verstehen, bevorzugt Mono- und Disaccharide, in denen gegebenenfalls eine oder mehrere Hydroxylgruppen durch in der Kohlenhydratchemie übliche Schutzgruppen aus der Reihe O-Alkyl, O-Alkyliden, O-Silyl, O-Stannyl, O-Stannyliden substituiert sind oder eine oder mehrere Hydroxylgruppen durch Aminogruppen, Acylamidogruppen, Azidogruppen, Nitrogruppen, Thiolgruppen, Niedrigalkoxygruppen oder Wasserstoff- oder Halogenatome ersetzt sein können und die Glycosylreste auch in Form der entsprechenden, gegebenenfalls mit den obigen funktionellen Gruppen substituierten Ulosen, Ulosederivate, Uronsäuren oder der Uronsäurederivate vorliegen können.

Die Glycosylreste Z in Formel (I) liegen erfindungsgemäß bevorzugt in der Pyranosyl- oder der Furanosylform vor, wobei die betreffenden Monosaccharid-, Disaccharid- oder Oligosaccharidreste bevorzugt aus Pentosen, Hexosen oder Heptosen aufgebaut sind.

Erfindungsgemäße Beispiele für Monosaccharidreste sind Glucopyranosyl-, Galactopyranosyl-, Mannopyranosyl-, Glucofuranosyl-, Ribofuranosyl-, Arabinopyranosyl- oder auch Lyxopyranosyl- oder auch D-Glycero-D-gluco-heptopyranosylreste. Als Beispiele für Di- und Oligosaccharidreste können genannt werden Maltosyl-, Maltotriosyl-, Maltotetraosyl-, Lactosyl-, Cellobiosyl- Melibiosyl- oder 6-O-(α- oder β-Ribofuranosyl)-Glucopyranosylreste, d.h. also Kohlenhydratsysteme, die aus Zuckern unterschiedlicher C-Zahl aufgebaut sind und bei denen die Zucker in der Pyranose und/oder Furanoseform vorliegen können. Die glykosidischen Bindungen zwischen den einzelnen Zuckerbausteinen können in der α- und/oder β-Form vorliegen und die glykosidische Verknüpfung der einzelnen Zuckerbausteine kann von einem anomeren Kohlenstoffatom ausgehend sowohl über die primäre OH-Gruppe als auch über eine der sekundären Hydroxylgruppen des als Aglykon fungierenden Saccharidteils erfolgen.

Als Beispiele für Mono-, Di- und Oligosaccharidreste, in denen eine oder mehrere OH-Gruppen durch Aminogruppen, Acylamidogruppen, Azidogruppen, Wasserstoff, Nitro, Thiolgruppen, Niedrigalkoxy oder Halogen ersetzt sind, seien genannt 2-Acetylamido-2-desoxygluco-pyranosyl, 2-Amino-2-desoxy-glucopyranosyl, 2-Caproylamido-2-desoxy-glucopyranosyl, 2-Lauroylamido-2-desoxy-glucopyranosyl, 2-Myristoylamido-2-desoxy-glucopyranosyl, 2-Palmitoylamido-2-desoxy-glucopyranosyl, 2-Stearoylamido-2-desoxy-glucopyranosyl, 4-Azido-4-desoxy-glucopyranosyl, 4-Stearoylamido-4-desoxy-glucopyranosyl 4-Dodecoylamido-4-desoxy-glucopyranosyl, 6-Hexadecanoyl-amido-6-desoxy-glucopyranosyl, 2,6-Diamino-2,6-

didesoxyglucopyranosyl, 6,6'-Diamino-6,6'-didesoxymaltosyl, 6-Amino-6,6'-didesoxylactosyl, 6-Desoxymannopyranosyl, 2-Desoxyribofuranosyl, Fucosyl, 5-Fluor-5-desoxy-ribofuranosyl, 6-0-Methylglucopyranosyl, 6-Desoxy-6-thio-glucopyranosyl, 3-Desoxy-3-nitrogalactopyranosyl.

Liegen die Glykosylreste in Form von Uronsäuren oder Uronsäurederivaten vor, so handelt es sich um Glykuronsäuren mit freier Carboxylgruppe oder mit durch Alkyl veresterer Carboxylgruppe oder um Glykuronamidderivate mit unsubstituiertem oder substituiertem Stickstoffatom. Beispiele für entsprechende Zucker sind Galacturonsäure, Glucuronsäuremethylester oder auch N-Dodecylglucuronamid.

Die Verbindungen der Formel (I) enthalten mehrere chirale C-Atome und liegen als optische reine Diastereomere oder als Diastereomerengemische vor.

Die erfindungsgemäßen Verbindungen der Formel (I) sind also Carbamidsäure-, Thiocarbamidsäure- oder Harnstoffderivate, die an dem durch den oben genannten Rest $R^1$ substituierten Stickstoffatom zusätzlich N-glycosidisch, d.h. über das anomere Kohlenstoffatom gebunden, einen einfachen oder modifizierten Mono-, Di- oder Oligosaccharidrest tragen.

Die Erfindung betrifft auch Verfahren zur Herstellung der Verbindungen gemäß Formel (I). Hierbei setzt man einen Zucker entweder in freier, d.h. ungeschützter Form, oder in Form geschützter, gegebenenfalls aktivierter Derivate zunächst um mit einer Aminoverbindung $R^1$-$NH_2$, entweder in freier Form oder in Form eines geeigneten Säureadditionssalzes, mit der vorstehend beschriebenen Bedeutung von $R^1$.

Die auf diese Weise erhaltenen Glycosylamine der Formel II

$$Z\text{-}NH\text{-}R^1 \qquad (II)$$

werden dann in einem zweiten Reaktionsschritt z. B. mit Halogenameisensäureestern der Formel III

$$Y\text{-}CO\text{-}O\text{-}R^2 \qquad (III)$$

umgesetzt. In Formel III steht Y für Halogen wie z. B. Chlor oder Brom und $R^2$ hat die oben angegebene Bedeutung. Auf diese Weise gelangt man zu den Urethanen gemäß Formel I, wobei X in Formel I für Sauerstoff steht.

Die Reaktion der im ersten Reaktionsschritt erhaltenen Glycosylamine der Formel II mit einem Thiokohlensäurehalogenid-S-ester der Formel IV

$$R^2\text{-}S\text{-}CO\text{-}Y \qquad (IV)$$

in der Y für Halogen wie z. B. Chlor steht und $R^2$ die oben angegebene Bedeutung hat, führt zu S-substituierten Thiocarbamaten der Formel I, wobei X in Formel I für ein Schwefelatom steht.

Werden die im ersten Reaktionsschritt erhaltenen Glycosylamine der Formel II im zweiten Reaktionsschritt mit einem organischen Isocyanat der Formel V

$$R^2\text{-}NCO \qquad (V)$$

umgesetzt, wobei $R^2$ die oben angeführte Bedeutung hat, so erhält man Harnstoffe gemäß Formel I, wobei in diesem Fall X in Formel I für NH steht.

Harnstoffe gemäß Formel I in denen X für NH oder N-Alkyl steht, erhält man, indem man Carbamate gemäß Formel I mit X = O und einem bevorzugt aromatischen, auf jeden Fall aktivierenden O-Substituenten wie z. B. p-Nitrophenyl aminolytisch mit primären Aminen $R^2$-$NH_2$ oder mit sekundären Aminen $R^2$-NH-Alkyl zum Harnstoff umsetzt.

Die auf diese Weise erhältlichen erfindungsgemäßen Verbindungen der Formel I werden dann, falls Schutzgruppen verwendet worden waren, entblockiert und, falls erforderlich, durch Chromatographie, Umkristallisieren, Extraktion o.ä. gereinigt.

In einer bevorzugten Ausführungsform des erfindungsgemäßen Verfahrens wird in an sich bekannter Weise (vgl. G.P. Ellis und J. Honeyman, Advances in Carbohydrate Chemistry 10, (1955) 95) der unblockierte Zucker Z-OH, wobei OH für die anomere Hydroxylgruppe steht, mit der in Formel I beschriebenen Bedeutung von Z, in einem geeigneten Lösungsmittel, oder auch ohne Lösungsmittel, gegebenenfalls in Gegenwart eines Katalysators bei Temperaturen zwischen 0° C und 80° C mit ein bis zehn Äquivalenten des betreffenden Amins R-$NH_2$ umgesetzt und man erhält gewöhnlich in hohen Ausbeuten nach Aufarbeitung die betreffenden Glycosylamine der Formel II als amorphe oder kristalline Feststoffe oder als zähe Sirupe.

Zur Darstellung der N-glycosylierten Carbamate gemäß Formel I, in der X für ein Sauerstoffatom steht, wird dann das Glycosylamin gemäß Formel II mit 1 bis 5 Äquivalenten eines Halogenameisensäureesters gemäß Formel III, bevorzugt Chlorameisensäureester, umgesetzt, wobei man in einem organischen oder wäßrig-organischen Lösungsmittel bei Temperaturen zwischen 0° C und 50° C, gegebenenfalls in Gegenwart einer anorganischen oder organischen Base, arbeitet und nach beendeter Reaktion das Reaktionsprodukt in üblicher Weise isoliert.

Zur Darstellung der N-glycosylierten und S-substituierten Thiocarbamate der Formel I, in der X für S steht, wird das Glycosylamin der Formel II mit 1 bis 10 Äquivalenten, bevorzugt 1 bis 3 Äquivalenten des S-substituierten Thiokohlensäurehalogenids der Formel IV in einem organischen Lösungsmittel zur Reaktion

gebracht, wobei die Reaktionstemperatur zwischen -10°C und +50°C, bevorzugt bei Temperaturen um 25°C liegt und die Reaktion gegebenenfalls in Gegenwart einer anorganischen oder organischen Base durchgeführt wird.

Zur Darstellung der N-glycosylierten Harnstoffe gemäß Formel I, in der X für NH steht, wird das Glycosylamin gemäß Formel II mit 1 bis 5, bevorzugt 1 bis 2 Äquivalenten, eines organischen Isocyanats gemäß Formel V zur Reaktion gebracht. Dazu arbeitet man bevorzugt in einem inerten organischen Lösungsmittel bei Temperaturen zwischen -20°C und 60°C, bevorzugt bei 20°C, gegebenenfalls unter Zusatz eines basischen Katalysators. Bei Katalysatorzusatz muß darauf geachtet werden, daß nicht parallel zur Umsetzung des Amins zum Harnstoff auch gegebenenfalls vorhandene Hydroxyl- oder Thiolgruppierungen mit dem Isocyanat reagieren.

Ein anderer Weg zur Darstellung der N-glycosylierten Harnstoffe gemäß Formel I, in der X für NH oder aber auch - abweichend von der vorstehenden Variante - für N-Alkyl stehen kann, besteht in der Aminolyse von N-Glycosylcarbamaten gemäß Formel I mit X = O, wobei $R^2$ für einen aktivierenden Esterrest, bevorzugt Phenyl oder p-Nitrophenyl steht. Die Aminolyse der aktiven Carbamate wird durchgeführt durch Umsetzung mit 1 bis 10 Äquivalenten, bevorzugt 1 bis 3 Äquivalenten, eines primären Amins $R^2$-$NH_2$ oder eines sekundären Amins $R^2$-NH-Alkyl in einem geeigneten organischen Lösungsmittel wie z. B. Tetrahydrofuran, Dioxan, N,N-Dimethylformamid bei Temperaturen zwischen 0°C und 120°C, bevorzugt 10°C bis 50°C, gegebenenfalls unter Zusatz von anorganischen oder organischen Basen wie z. B. Kaliumcarbonat, Triethylamin oder Pyridin.

Für den Fall, daß im Kohlenhydratrest Z eine oder mehrere freie Aminogruppen vorhanden sind, werden diese vor der Amsetzung mit dem Amin $R^1$-$NH_2$, in an sich bekannter weise mit einer Aminoschutzgruppe versehen.

Als Aminoschutzgruppen kommen solche in der Zucker- und Peptidchemie üblicherweise verwendeten Gruppen in Frage (siehe z. B. Houben-Weyl, Methoden der organischen Chemie, Band XV, Georg Thieme Verlag, Stuttgart, 1974), die einerseits unter den gegebenen Reaktionsbedingungen stabil sind, die andererseits aber nach der Herstellung des betreffenden N-Glykosids und dessen anschließender Umsetzung mit den Verbindungen der Formeln III, IV oder V so selektiv wieder abgespalten werden können, daß das gewünschte Endprodukt der Formel I erhalten wird, d.h. ohne daß die im Endprodukt der Formel I enthaltenen Carbamat-, Thiocarbamat- oder Harnstoffgruppen gespalten werden. Bevorzugte Beispiele sind Acylgruppen vom Typ

```
-C-B
 ||
 O
```

wobei B Trichlormethyl oder Trifluormethyl bedeutet der vom Typ

```
-C-O-E
 ||
 O
```

wobei E z. B. für Ethyl, Trichlorethyl, Benzyl oder tertiär-Butyl steht oder auch Sulfenylgruppen vom Typ

-S-G

wobei G für Phenyl, substituiertes Phenyl oder Di- oder Triphenylmethyl steht und "substituiertes Phenyl" einen Phenylrest bezeichnet, der durch einen bis drei Substituenten aus der Reihe Nitro, Niedrigalkyl oder der durch 1 bis 5 Halogenatome, vorzugsweise Chloratome, substituiert ist. Als Beispiele seien der 2,4,5-Trichlorphenylsulfenyl und der o-Nitrophenylsulfenylrest genannt.

Die Einführung dieser Schutzgruppen in Aminoverbindungen und ihre spätere Abspaltung zur Freisetzung der gewünschten Aminogruppen sind bekannt und beispielsweise in der oben zitierten Literaturstelle beschrieben.

In einer anderen Ausführungsform des Verfahrens zur Herstellung von solchen Produkten der Formel I in denen im Glycosylrest Z eine oder mehrere freie Aminogruppen vorhanden sind, setzt man Zuckerderivate Z-OH als Ausgangsprodukte ein, in welchen die Aminogruppe oder die Aminogruppen zunächst in Form von Azidoresten, d.h. also in verkappter Form vorliegen. Im abschließenden Schritt der Herstellung der Verbindungen der Formel I werden diese Azidogruppen reduktiv in an sich bekannter, Weise in Aminogruppen umgewandelt, wobei darauf geachtet wird, daß solche Reduktionsmittel verwendet werden, die andere gegebenenfalls im Molekül vorhandene reduktionsempfindliche Gruppen nicht angreifen.

Entsprechende Azidozucker und deren Herstellung sind bekannt (siehe z. B. Methods in Carbohydrate Chemistry Vol. I, New York a. London 242 bis 246, Academic Press, 1962). Zur Reduktion können verwendet werden Hydriddonatoren wie z. B. Natriumboranat oder Lithiumalanat, katalytisch erregter Wasserstoff oder auch Triphenylphosphin in Methanol/Ammoniak/Pyridin oder auch Schwefelwasserstoff oder Mercaptane in protischen Solventien.

Als Lösungsmittel können alle gängigen organischen Solventien, vorzugsweise niedrige Alkanole, oder aber auch Wasser oder wäßrige Alkanole verwendet werden.

Die Reaktionen werden gegebenenfalls unter Zusatz von organischen Säuren wie z. B. Essigsäure oder

anorganischen Säuren wie z. B. Schwefelsäure oder unter Zusatz organischer Basen wie z. B. Pyridin oder anorganischen Basen wie z. B. Ammoniak durchgeführt. Man arbeitet bei Temperaturen zwischen 0 und 120° C, vorzugsweise 10° C bis 40° C, gegebenenfalls unter erhöhtem Druck und/oder Inertgas.

Für den Fall, daß in den erfindungsgemäßen Endprodukten der Formel I im Kohlenhydratteil Z eine oder mehrere OH-Gruppen durch eine oder mehrere Acylamidogruppen ersetzt sind, werden die Zucker Z-OH von vornherein in Form der entsprechenden Acylamidozucker eingesetzt. Die Acylamidozucker werden dann am anomeren Zentrum mit den obigen angegebenen Aminen zunächst zu den entsprechenden Acylamidoglycosylaminen umgesetzt und im zweiten Reaktionsschritt an der C-1 Aminogruppe des Zuckerteils mit Halogenkohlensäureestern der Formel III, bzw. mit Thiokohlensäurechlorid-S-estern der Formel IV, bzw. mit Isocyanaten der Formel V zu den N-(Acylamidoaldosyl)-carbamaten bzw. -thiocarbamaten bzw. -harnstoffen gemäß Formel I umgesetzt.

Ferner ist es auch möglich, die Verbindungen der Formel I in der Z für einen mit einer oder mehreren Acylamidogruppen substituierten Zuckerrest steht, zu erhalten, indem man in Derivaten der Formel I, in der Z zunächst einen durch eine oder mehrere temporäre Amino-Schutzgruppen vom vorstehend beschriebenen Typ

$-\underset{\overset{\|}{O}}{C}-B$, $-\underset{\overset{\|}{O}}{C}-O-E$ oder $-S-G$

blockierten Aminozucker darstellt, die temporäre Amino-Schutzgruppe nach den üblichen Methoden abspaltet zu den entsprechenden N-(Aminodesoxyglycosyl)-urethanen, -thiocarbamaten bzw. -harnstoffen gemäß Formel I und die freie Aminogruppe am Zuckerring dann nach den üblichen Methoden der organischen Chemie acyliert zu N-(Acylamidodesoxyglycosyl)-urethanen bzw. -thiocarbamaten bzw. -harnstoffen.

Der erste Verfahrensschritt bei der Herstellung der erfindungsgemäßen Verbindungen der Formel I ist somit die Umsetzung eines Zuckers Z-OH, mit einem Amin des Typs $R^1-NH_2$ am anomeren Kohlenstoffatom unter Wasserabspaltung zu dem betreffenden Glykosylamin.

Amine $R^1-NH_2$, die bei Raumtemperatur flüssig sind, können mit dem Zucker direkt, d.h. ohne Lösungsmittel umgesetzt werden. Hierbei arbeitet man bei Temperaturen zwischen 0° C und 100° C, vorzugsweise bei 25° C bis 70° C. Als Katalysatoren sind Mineralsäuren wie z. B. Salzsäure, Schwefelsäure oder Salpetersäure oder kurzkettige Carbonsäuren wie Essigsäure oder Propionsäure geeignet, die man in Mengen von 0,001 bis 0,05 Äquivalenten einsetzt.

In jedem Fall ist es möglich, und bei Aminen $R^1-NH_2$, die bei Raumtemperatur fest sind auch zu bevorzugen, die Herstellung der Glykosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsprodukt in ihm lösen.

In Frage kommen Alkohole wie Methanol, Ethanol, Propanol-1 und Propanol-2, Ether wie Tetrahydrofuran oder Dioxan, sowie auch Dimethylformamid, wobei - außer bei der Verwendung der Alkohole - der Zusatz von Wasser zu bevorzugen ist. Darüber hinaus ist vorzugsweise bei kurzkettigen Aminen $R^1-NH_2$ auch Wasser allein als Lösungsmittel geeignet. Es kann auch von Vorteil sein, die Alkanole im Gemisch mit Wasser zu verwenden.

Die Reaktionstemperaturen bei Verwendung von Lösungsmitteln bei der Herstellung der Glykosylamine liegen zwischen -10° C und 120° C, vorzugsweise zwischen 30° C und 70° C.

Das betreffende Verdünnungsmittel kann wahlweise vor oder während der Reaktion zugegeben werden. Bei langkettigen Aminen $R^1-NH_2$ ist eine Zugabe vor der Reaktion zu bevorzugen.

Die wie vorstehend beschrieben hergestellten Glykosylamine kristallisieren entweder direkt oder nach Abkühlen aus und können durch Zusatz geeigneter, vorzugsweise weniger polarer Hilfslösungsmittel wie Aceton, Diethylether, Cyclohexan, Essigester oder Petrolether, gegebenenfalls unter Kühlung, ausgefällt oder zur Kristallisation gebracht werden; gegebenenfalls vorhandenes überschüssiges Amin $R^1-NH_2$ kann durch Waschen oder Umkristallisieren des Produktes auf an sich bekannter Weise entfernt werden.

Der zweite Verfahrensschritt bei der Herstellung der erfindungsgemäßen Verbindungen der Formel I ist die selektive Umsetzung eines wie vorstehend beschrieben erhaltenen Glycosylamins der Formel II mit Kohlensäurederivaten der Formeln III, IV oder V.

Diese Kohlensäurederivate werden vorzugsweise in Gegenwart eines Verdünnungsmittels mit den Glycosylaminen umgesetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern. Man kann sowohl in organischen Lösungsmitteln wie Ethern, z. B. Tetrahydrofuran und Dioxan, oder Alkoholen z. B. Ethanol und Propanol, oder Ketonen, z. B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Umsetzungen mit den Kohlensäurederivaten können in Gegenwart basischer Hilfsstoffe durchgeführt werden. Verwendet werden können alle in der organischen Synthese üblichen basischen Verbindungen wie z. B. tertiäre aliphatische oder auch aromatische Amine oder aber Alkali- und Erdalkalihydroxide bzw. -carbonate wie Natronlauge, Natriumcarbonat oder Calciumcarbonat.

Die auf diese Weise erhaltenen Carbamate, Thiocarbamate bzw. Harnstoffe werden nach an sich bekannten

Verfahren in Form von kristallinen oder amorphen Feststoffen oder als zähe Sirupe isoliert und, wenn notwendig, durch Umkristallisation, Chromatographie, Extraktion usw. gereinigt.

Im Falle von Verbindungen mit geschützten Aminogruppen im Glykosylteil werden die Schutzgruppen in an sich bekannter Weise abgespalten.

Das folgende Formelschema soll eine der bevorzugten Ausführungsformen der erfindungsgemäßen Darstellung von Verbindungen der Formel I beispielhaft erläutern.

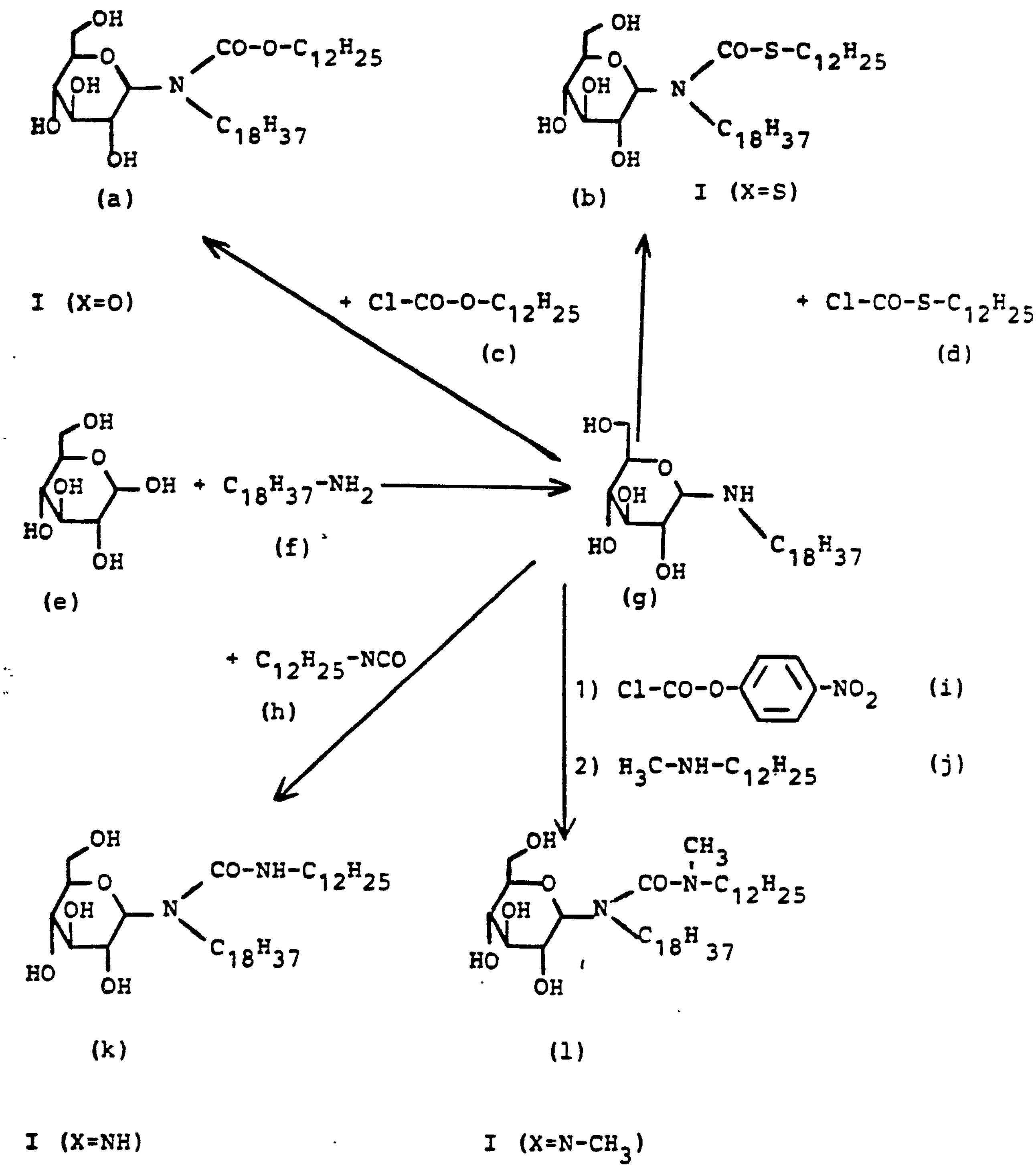


(a) I (X=O)

(b) I (X=S)

(e)

(g)

(k) I (X=NH)

(l) I ($X=N-CH_3$)

Glucose (e) wird mit Stearylamin (f) zum Glucosylamin (g) umgesetzt. Im zweiten Reaktionsschritt wird das Glycosylamin (g) mit Chlorameisensäuredodecylester (c) zum Carbamat (a) [= I (X = O)] oder mit Thiokohlensäurechlorid-S-dodecylester (d) zum S-Dodecyl-thiocarbamat (b) [= I (X = S)] oder mit Dodecylisocyanat (h) zum Harnstoff (k) [= I (X = NH)I] oder nacheinander mit Chlorameisensäure-p-nitrophenylester (i) und N-Methyl-N-dodecylamin (j) zum Harnstoff (l) [= I (X = $N-CH_3$)] umgesetzt.

Zum Gegenstand der Erfindung gehören auch Salze der Verbindungen der Formel I. Dabei handelt es sich in erster Linie um üblicherweise pharmazeutisch verwendbare, nicht-toxische Salze, z. B. Alkalimetall- oder Ammoniumsalze, oder Chloride oder Acetate.

Die Verbindungen der Erfindung weisen eine ausgeprägte Abwehr-steigernde Wirkung auf. Es wurde gefunden, daß die Verbindungsklasse die Antikörpersynthese des Immunsystems Antigen-spezifisch steigert und darüber hinaus die unspezifische wirtseigene Abwehr verstärkt. Diese Ergebnisse wurden anhand der nachfolgenden Versuchsanordnung erhalten.

Steigerung der primären humoralen Immunität in vitro gegen Schaferythrozyten (SE).

Es ist experimentell möglich, die Entwicklung einer humoralen Immunantwort mit heterologen roten Blutzellen durch primäre Immunisation von Maus-Milzzellen in Suspensionskulturen in vitro einzuleiten (R. I. Mishell und R. W. Dutton, J. Exp. Med. 126, (1967) 423). Hierzu werden Balb/c Maus-Milzzellen für 5 Tage in Gegenwart von Antigen (SE) und der Testsubstanz kultiviert. Die Zellen werden geerntet, gewaschen und zusammen mit dem Antigen und Komplement in semisolidem Agar ausplattiert und für 2 Stunden bei 37°C inkubiert (N. K. Jerne, A. A. Nordin und C. Henry, "Cell Bound Antibodies", eds. Amos and Koprowski, Wistar Inst. Fress, Philadelphia, USA, (1963) pp 109). Die Antigen-Sensibilisierung von Maus-Lymphozyten in der Primärkultur resultiert in der Synthese und Freisetzung von Antikörper. Die spezifischen, sezernierten Antikörper binden sich an das SE-Antigen und lysieren diese Zellen durch die Gegenwart des Komplements (Plague-Bildung). Substanzen der vorliegenden Verbindungsklasse sind in der Lage, dosisabhängig im Bereich 3 - 100 µg/ml die Zahl der Antikörper-bildenden Zellen zu steigern (Tabelle 1).

**Tabelle 1**

Wirkung von verschiedenen, ausgewählten N-glykosylierten Substanzen der vorliegenden Verbindungsklasse

| Substanzen | Antikörper-sezernierende Zellen/Kultur in Abhängigkeit von der Dosis (µg/ml) | | | | |
|---|---|---|---|---|---|
| | Beispiel-Nr. | 0 | 3 | 10 | 30 |
| 100 | | | | | |
| | 28 | 4460 | 4850 | 6300 | 16760 |
| 18560 | 10 | 1590 | 2920 | 6480 | 7920 |
| 11880 | 9 | 1560 | 1020 | 1570 | 7940 |
| 9720 | 15 | 485 | 725 | 1830 | 1950 |
| 6480 | 11 | 1190 | 410 | 1020 | 3780 |
| 6080 | | | | | |

Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s.c.) immunisiert. Bei suboptimaler Antigenstimulation wurde nur eine geringe Zahl von Lymphozyten der Tiere zur Antikörpersynthese angeregt. Die zusätzliche Behandlung der Tiere mit Verbindungen der genannten Beispiele der vorliegenden Erfindung ist in der Lage, bei einer einmaligen Applikation von 10 - 30 mg/kg subcutan den Antikörpertiter im Serum der Tiere signifikant zu steigern. Die Antikörpertiterbestimmung erfolgt durch indirekte Hämagglutination am Tag 10. Der Effekt der Behandlung wird durch den geometrischen Mittelwert der $log_2$-Titer ausgedrückt.

Der immunstimulierende Effekt der genannten Verbindungen ist im Gegensatz zu anderen, z. B. bakteriellen Immunstimulantien wie LPS aus Gram-negativen Bakterien Antigen-abhängig, d.h. die Substanzen bewirken überraschenderweise nur in Verbindung mit einem antigenen Reiz (hier SE oder Ovalbumin) die Induktion der Antikörpersynthese. Sie haben im Gegensatz zu den erwähnten konventionellen Immunstimulantien keine mitogenen Eigenschaften.

Verträglichkeit

Obwohl Verbindungen der beschriebenen Art ihre potenzierende Wirkung an der Maus beispielsweise bereits nach einer Einzeldosis von 10 mg/kg i.p., oder peroral entfalten, werden auch bei Applikation von 100 mg/kg keine toxischen Effekte beobachtet. Die genannten Stoffe verfügen deshalb über eine gute Verträglichkeit.

Die erfindungsgemäßen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z. B. bei chronischen und akuten Infektionen oder bei selektiven (antigenspezifischen) immunologischen Defekten,

sowie bei angeborenen, aber auch erworbenen allgemeinen (d.h. nicht antigenspezifisch) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen erhöhen die Überlebensrate im Tiermodell der systemischen Mäusecandidose und der akuten bakteriellen Infektion.

Versuchsbeschreibung

Mäuse vom Typ SPF-CFW 1 wurden intravenös mit 2 - 6 x $10^5$ logarithmisch wachsenden Zellen von Candida albicans, suspendiert in physiologischer Kochsalzlösung, infiziert. Beginnend mit dem 3. Tag post infectionem werden bei unbehandelten Kontrolltieren die ersten Krankheitssymptome erkennbar. Bis zum 5. Tag sterben die ersten Tiere an akutem Nierenversagen und bis zum 14. Tag post infectionem sind in der Regel mehr als 80 % der unbehandelten Tiere gestorben. In diesem Test sind die erfindungsgemäßen Verbindungen krankheitsverzögernd wirksam. Eine signifikante krankheitsverzögernde Wirkung wurde erreicht, wenn z. B. die Substanz gemäß Beispiel 24 jeweils einmal 24 Stunden vor der Infektion in Konzentrationen von 1 - 50 mg/kg Körpergewicht intraperitoneal (i.p.) verabreicht wurde.

Bei behandelten Tieren wurde eine statistisch signifikante Verlängerung der Überlebenszeit im Vergleich zu den unbehandelten Kontrollen beobachtet. Etwa 50 % der behandelten Tiere überlebten einen Beobachtungszeitraum von 14 Tagen verglichen mit etwa 20 % unbehandelter Kontrolltiere.

Die erfindungsgemäßen Verbindungen können allein als Prophylaktikum, zur Bekämpfung bestehender Infektionen oder in Kombination mit einer antibiotischen Therapie zur Steigerung der therapeutischen Wirkung von Antibiotika und Chemotherapeutika (z. B. Penicilline, Cephalosporine, Aminoglykoside etc.) bei infizierten Menschen und Tieren verwendet werden.

Es wurde gefunden, daß Infektionen der Maus mit pathogenen Keimen, die innerhalb von 24 - 48 Stunden zum Tod der Versuchstiere führen, durch eine prophylaktische Behandlung - bevorzugt intraperitoneal - mit 1 - 80 mg/kg der erfindungsgemäßen Verbindungen therapiert werden können. Dies trifft für eine ganze Reihe grampositiver (z. B. Staphylokokken) und gramnegativer (z. B. E. coli, Klebsiella, Proteus, Pseudomonas) Krankheitserreger zu. Diese Aufzählung ist beispielhaft und keineswegs beschränkend aufzufassen. So überleben z. B. Mäuse, die mit dem pathogenen Stamm Klebsiella 63 infiziert worden waren, nach Behandlung (z. B. 18 Stunden vor Infektion) mit 10 - 40 mg/kg der erfindungsgemäßen Verbindung gemäß Beispiel 12, 13, 19, 22 oder 24, zu 40 bis 100 % diese Infektion, während von den unbehandelten Kontrolltieren nur 0 bis 30 % überlebten.

In einem weiteren Versuchsmodell konnte gezeigt werden, daß die therapeutische Wirksamkeit von Antibiotika durch die erfindungsgemäßen Verbindungen gesteigert werden kann. So wurden Mäuse mit dem Stamm Pseudomonas W. infiziert. Diese Infektion führt bei den meisten Kontrolltieren innerhalb 24 Stunden zum Tode. Eine weitere Gruppe wurde mit 4 mg/kg Sisomicin 30 Stunden post infectionem behandelt. Es konnte gezeigt werden, daß in der Versuchsgruppe, die mit den erfindungsgemäßen Verbindungen (Beispiele siehe oben) 18 Stunden vor Infektion behandelt worden waren, die therapeutische Wirksamkeit des Sisomicins entscheidend verbessert werden konnte.

Die pharmazeutischen Präparate der vorliegenden Erfindung sind vorzugsweise Tabletten oder Gelatinekapseln, welche die Wirkstoffe zusammen mit Verdünnungsmitteln, z. B. Lactose, Dextrose, Saccharose, Mannit, Sorbit, Cellulose und/oder Schmiermitteln, z. B. Kieselerde, Talk, Stearinsäure oder Salze davon, wie Magnesium- oder Calciumstearat, und/oder Polyethylenglykol, enthalten. Tabletten enthalten ebenfalls Bindemittel, z. B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z. B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1 % bis etwa 75 % insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z. B. bakterielle, virale und parasitäre) und malignen Tumoren verwendet werden. Sie können ebenfalls als Adjuvantien bei der Vakzinierung, bei der Stimulierung von Phagocytose, bei der Dysregulierung des Abwehr- und Immunsystems verwendet werden.

## Beispiele

### Beispiel 1

N-(D-Glucopyranosyl)-dodecylamin
18 g D-Glucose werden in 50 ml Ethanol bei 70°C gerührt, dann fügt man 18,5 g Dodecylamin zu, heizt noch bis zur klaren Lösung, läßt auf Raumtemperatur abkühlen und saugt noch 20 Stunden von den ausgefallenen Kristallen ab. Man wäscht mit Ethanol und Ether und trocknet im Vakuum.

Elementaranalyse

| | | | |
|---|---|---|---|
| ber.: | C 62,2 % | H 10,6 % | N 4,0 % |
| gef.: | C 62,2 % | H 10,6 % | N 4,2 % |

### Beispiel 2

N-(D-Glucopyranosyl)-octadecylamin
Herstellung analog Beispiel 1 aus 11 g Glucose und 20 g Octadecylamin

Elementaranalyse

| | | | |
|---|---|---|---|
| ber.: | C 66,8 % | H 11,4 % | N 3,2 % |
| gef.: | C 66,9 % | H 11,1 % | N 3,4 % |

### Beispiel 3

N-(D-Galactopyranosyl)-tetradecylamin
Herstellung analog Beispiel 1 aus 18 g Galactose und 20 g Tetradecylamin

Elementaranalyse

| | | | |
|---|---|---|---|
| ber.: | C 64,0 % | H 10,9 % | N 3,7 % |
| gef.: | C 64,1 % | H 11,0 % | N 3,8 % |

### Beispiel 4

N-(D-Galactopyranosyl)-octadecylamin
Herstellung analog Beispiel 1 aus 11 g Galactose und 20 g Octadecylamin

Elementaranalyse

| | | | |
|---|---|---|---|
| ber.: | C 66,8 % | H 11,4 % | N 3,2 % |
| gef.: | C 66,9 % | H 11,2 % | N 3,4 % |

### Beispiel 5

N-(D-Mannopyranosyl)-dodecylamin
Herstellung analog Beispiel 1 aus 18 g Mannose und 19 g Dodecylamin

Elementaranalyse

| | | | |
|---|---|---|---|
| ber.: | C 62,2 % | H 10,7 % | N 4,0 % |
| gef.: | C 62,3 % | H 10,8 % | N 4,2 % |

**Beispiel 6**

N-(D-Mannopyranosyl)-octadecylamin
Herstellung analog Beispiel 1 aus 22 g Mannose und 40 g Octadecylamin

Elementaranalyse

| | | | |
|---|---|---|---|
| ber.: | C 66,8 % | H 11,4 % | N 3,2 % |
| gef.: | C 66,9 % | H 11,2 % | N 3,1 % |

**Beispiel 7**

N-(D-Ribopyranosyl)-dodecylamin
Herstellung analog Beispiel 1 aus 15 g D-Ribose und 18 g Dodecylamin

Elementaranalyse

| | | | |
|---|---|---|---|
| ber.: | C 64,4 % | H 11,0 % | N 4,4 % |
| gef.: | C 64,4 % | H 11,2 % | N 4,6 % |

**Beispiel 8**

N-(2-Acetamido-2-desoxy-D-glucopyranosyl)-dodecylamin
15 g N-Acetylglucosamin und 19 g Dodecylamin werden für 3 h in 50 ml Ethanol auf 80°C unter Rühren erhitzt. Dann wird heiß vom Ungelösten abfiltriert, das Filtrat abgekühlt, das ausgefallene Produkt abgesaugt und mit Ethanol und Ether nachgewaschen. Der Filterrückstand wird im Vakuum getrocknet.

Elementaranalyse

| | | | |
|---|---|---|---|
| ber.: | C 61,9 % | H 10,3 % | N 7,2 % |
| gef.: | C 62,1 % | H 10,3 % | N 7,4 % |

**Beispiel 9**

N-(D-Glucopyranosyl)-N-dodecyl-dodecylurethan
7,0 g der Verbindung aus Beispiel 1 werden in 50 ml Tetrahydrofuran suspendiert und mit 10 g Kaliumcarbonat gerührt. Es werden 6,7 g Chlorameisensäuredodecylester zugegeben und bis zur Beendigung der Reaktion (Dünnschichtchromatographische Kontrolle) weitergerührt. Der Ansatz wird mit 50 ml Tetrahydrofuran verdünnt, filtriert und das Filtrat eingedampft und säulenchromatographisch gereinigt (Laufmittel Toluol/Isopropanol 10 : 1).
$\alpha_D$ = 4,7° (c = 1,0 in Tetrahydrofuran)

**Beispiel 10**

N-(D-Glucopyranosyl)-N-dodecyl-tetradecylurethan
Herstellung analog Beispiel 9 aus 7,0 g der Verbindung aus Beispiel 1 und 7,4 g Chlorameisensäure-tetradecylester
$\alpha_D$ = 4,5° (c = 0,95 in Tetrahydrofuran)

**Beispiel 11**

N-(D-Glucopyranosyl)-N-dodecyl-pentadecylurethan
Herstellung analog Beispiel 9 aus 7,0 g der Verbindung aus Beispiel 1 und 7,8 g Chlorameisensäure-pentadecylester.
$\alpha_D$ = 4,5° (c = 1,1 in Tetrahydrofuran)

**Beispiel 12**

N-(D-Glucopyranosyl)-N-dodecyl-octadecylurethan
Herstellung analog Beispiel 9 aus 7,0 g der Verbindung aus Beispiel 1 und 8,9 g Chlorameisensäureoctadecylester.

**Beispiel 13**

N-(D-Glucopyranosyl)-N-octadecyl-decylurethan
Herstellung analog Beispiel 9 aus 8,6 g der Verbindung aus Beispiel 2 und 5,9 g Chlorameisensäure-decylester.
$\alpha_D$ = 3,9° (c = 1,01 in Tetrahydrofuran).

**Beispiel 14**

N-(D-Glucopyranosyl)-N-octadecyl-dodecylurethan
Herstellung analog Beispiel 9 aus 8,6 g der Verbindung aus Beispiel 2 und 6,7 g Chlorameisensäure-dodecylester.
$\alpha_D$ = 4,0° (c = 1,4 in Tetrahydrofuran)

**Beispiel 15**

N-(D-Glucopyranosyl)-N-octadecyl-tetradecylurethan
Herstellung analog Beispiel 9 aus 8,6 g der Verbindung aus Beispiel 2 und 7,4 g Chlorameisensäuretetradecylester.

**Beispiel 16**

N-(D-Glucopyranosyl)-N-octadecyl-pentadecylurethan
Herstellung analog Beispiel 9 aus 8,6 g der Verbindung aus Beispiel 2 und 7,8 g Chlorameisensäurepentadecylester.

**Beispiel 17**

N-(D-Glucopyranosyl)-N-octadecyl-octadecylurethan
Herstellung analog Beispiel 9 aus 8,6 g der Verbindung aus Beispiel 2 und 8,9 g Chlorameisensäureoctadecylester.

**Beispiel 18** (gestrichen)

**Beispiel 19**

N-(D-Glucopyranosyl)-N-octadecyl-(octylthioethyl)-urethan
Herstellung analog Beispiel 9 aus 8,6 g der Verbindung aus Beispiel 2 und 6,8 g Chlorameisensäureoctylthioethylester.

**Beispiel 20** (gestrichen)

**Beispiel 21**

N-(D-Glucopyranosyl)-N-dodecyl-(4-tert.-butylcyclohexyl)-urethan
Herstellung analog Beispiel 9 aus 7,0 g der Verbindung aus Beispiel 1 und 5,9 g Chlorameisensäure-(4-tert.-butyl-cyclohexyl)-ester.

**Beispiel 22**

N-(D-Glucopyranosyl)-N-dodecyl-(4-(1,1,3,3-tetramethylbutyl)-cyclohexyl)-urethan
Herstellung analog Beispiel 9 aus 7,0 g der Verbindung aus Beispiel 1 und 7,4 g Chlorameisensäure-(4-(1,1,3,3-tetramethylbutyl)-cyclohexyl)-ester.

**Beispiel 23**

N-(D-Glucopyranosyl)-N-octadecyl-(4-tert.-butylcyclohexyl)-urethan
Herstellung analog Beispiel 9 aus 8,6 g der Verbindung aus Beispiel 2 und 5,9 g Chlorameisensäure-(4-tert.-butylcyclohexyl)-ester.

**Beispiel 24**

N-(D-Glucopyranosyl)-N-octadecyl-(4-(1,1 3 3-tetramethylbutyl)-cyclohexyl)-urethan
Herstellung analog Beispiel 9 aus 8,6 g der Verbindung aus Beispiel 2 und 7,4 g Chlorameisensäure-(4-(1,1,3,3-tetramethylbutyl)-cyclohexyl)-ester.

**Beispiel 25**

N-(D-Galactopyranosyl)-N-tetradecyl-octadecylurethan
Herstellung analog Beispiel 9 aus 7,5 g der Verbindung aus Beispiel 3 und 8,9 g Chlorameisensäureoctadecylester.

**Beispiel 26**

N-(D-Galactopyranosyl)-N-octadecyl-dodecylurethan
Herstellung analog Beispiel 9 aus 8,6 g der Verbindung aus Beispiel 4 und 6,7 g Chlorameisensäuredodecylester.

**Beispiel 27**

N-(D-Galactopyranosyl)-N-octadecyl-octadecylurethan
Herstellung analog Beispiel 9 aus 8,6 g der Verbindung aus Beispiel 4 und 8,9 g Chlorameisensäureoctadecylester.

**Beispiel 28**

N-(D-Galactopyranosyl)-N-octadecyl-(4-tert.-butylcyclohexyl)-urethan
Herstellung analog Beispiel 9 aus 8,6 g der Verbindung aus Beispiel 4 und 5,9 g Chlorameisensäure-(4-tert.-butylcyclohexyl)-ester.

**Beispiel 29**

N-(D-Mannopyranosyl)-N-dodecyl-octadecylurethan
Herstellung analog Beispiel 9 aus 7,0 g der Verbindung aus Beispiel 5 und 8,9 g Chlorameisensäureoctadecylester.

**Beispiel 30**

N-(D-Mannopyranosyl)-N-octadecyl-dodecylurethan
Herstellung analog Beispiel 9 aus 8,6 g der Verbindung aus Beispiel 6 und 6,7 g Chlorameisensäuredodecylester.

**Beispiel 31**

N-(D-Mannopyranosyl)-N-octadecyl-tetradecylurethan
Herstellung analog Beispiel 9 aus 8,6 g der Verbindung aus Beispiel 6 und 7,4 g Chlorameisensäuretetradecylester.

**Beispiel 32**

N-(D-Mannopyranosyl)-N-dodecyl-(4-tert.-butylcyclohexyl)-urethan
Herstellung analog Beispiel 9 aus 7,0 g der Verbindung aus Beispiel 5 und 5,9 g Chlorameisensäure-(4-tert.-butylcyclohexyl)-ester.

**Beispiel 33**

N-(D-Mannopyranosyl)-N-octadecyl-(4-(1,1,3,3-tetramethylbutyl)-cyclohexyl)-urethan
Herstellung analog Beispiel 9 aus 8,6 g der Verbindung aus Beispiel 6 und 5,9 g Chlorameisensäure-(4-(1,1,3,3-tetramethylbutyl)-cyclohexyl)-ester.

**Beispiel 34** (gestrichen)

**Beispiel 35** (gestrichen)

**Beispiel 36**

N-(D-Ribopyranosyl)-N-dodecyl-dodecyl-urethan
Herstellung analog Beispiel 9 aus 6,4 g der Verbindung aus Beispiel 7 und 6,7 g Chlorameisensäuredodecylester.

**Beispiel 37**

N-(D-Ribopyranosyl)-N-dodecyl-octadecyl-urethan
Herstellung analog Beispiel 9 aus 6,4 g der Verbindung aus Beispiel 7 und 8,9 g Chlorameisensäureoctadecylester.

**Beispiel 38**

N-(D-Ribopyranosyl)-N-dodecyl-(4-tert.-butyl-cyclohexyl)-urethan
Herstellung analog Beispiel 9 aus 6,4 g der Verbindung aus Beispiel 7 und 5,9 g Chlorameisensäure-(4-tert.-butylcyclohexyl)-ester.

**Beispiel 39**

N-(2-Acetamido-2-desoxy-D-glucopyranosyl)-N-dodecyl-dodecylurethan
Herstellung analog Beispiel 9 aus 7,8 g der Verbindung aus Beispiel 8 und 6,7 g Chlorameisensäure-dodecylester.

**Beispiel 40**

N-(2-Acetamido-2-desoxy-D-glucopyranosyl)-N-dodecyl-tetradecylurethan
Herstellung analog Beispiel 9 aus 7,8 g der Verbindung aus Beispiel 8 und 7,4 g Chlorameisensäure-tetradecylester.

**Beispiel 41** (gestrichen)

**Beispiel 42**

N-(D-Glucopyranosyl)-N-dodecyl-N'-dodecyl-harnstoff
7,0 g der Verbindung aus Beispiel 1 werden in 150 ml Methanol suspendiert. Dazu werden 4,2 g Dodecylisocyanat, gelöst in 20 ml Tetrahydrofuran, getropft. Nach ca. 4 h wird im Vakuum eingedampft und säulenchromatographisch gereinigt (Laufmittel Dichlormethan/Methanol 15 : 1).
$\alpha_D$ = 13,4° (c = 1,11 in Tetrahydrofuran)

**Beispiel 43**

N-(D-Glucopyranosyl)-N-dodecyl-N'-tetradecyl-harnstoff
Herstellung analog Beispiel 42 aus 7,0 g der Verbindung aus Beispiel 1 und 4,8 g Tetradecylisocyanat.

**Beispiel 44**

N-(D-Glucopyranosyl)-N-dodecyl-N'-octadecyl-harnstoff
Herstellung analog Beispiel 42 aus 7,0 g der Verbindung aus Beispiel 1 und 5,9 g Octadecylisocyanat.
$\alpha_D$ = 13,2° (c = 0,97 in Tetrahydrofuran)

**Beispiel 45**

N-(D-Glucopyranosyl)-N-octadecyl-N'-tetradecyl-harnstoff
Herstellung analog Beispiel 42 aus 8,6 g der Verbindung aus Beispiel 2 und 4,8 g Tetradecylisocyanat.

**Beispiel 46** (gestrichen)

**Beispiel 47**

N-(D-Glucopyranosyl)-N-dodecyl-(4-nitrophenyl)-urethan
Herstellung analog Beispiel 9 aus 17,4 g der Verbindung aus Beispiel 1 und 10,1 g Chlorameisensäure-(4-nitrophenyl)-ester.

**Beispiel 48**

N-(D-Glucopyranosyl)-N-dodecyl-N'-methyl-N'-octadecyl-harnstoff
3,1 g der Verbindung aus Beispiel 47 werden in 50 ml Dimethylformamid gelöst und mit 5,0 g Kaliumcarbonat versetzt. Es werden 1,8 g N-Methyl-octadecylamin zugegeben und auf 50°C erwärmt. Nach Beendigung der Reaktion wird auf Raumtemperatur abgekühlt, filtriert, das Filtrat im Vakuum eingedampft und säulenchromatographisch gereinigt (Laufmittel Toluol/Isopropanol 10 : 1)
$\alpha_D$ = 8,5° (c = 1 in Tetrahydrofuran)

**Beispiel 49**

N-(D-Glucopyranosyl)-N-dodecyl-S-octadecyl-thiocarbamat
7,0 g der Verbindung aus Beispiel 1 werden in 100 ml Tetrahydrofuran suspendiert und mit 1,1 g Triethylamin versetzt. Es werden 7,0 g Thiokohlensäurechlorid-S-octadecylester zugegeben. Nach Beendigung der Reaktion wird mit 100 ml Methanol/Triethylamin 1 : 1 1 Stunde nachgerührt, filtriert und das Filtrat im Vakuum eingeengt. Säulenchromatographische Reinigung (Laufmittel Toluol/Isopropanol 10 : 1).
$\alpha$ = + 8,6° (c = 1,05 in Tetrahydrofuran)

**Beispiel 50** (gestrichen)

**Beispiel 51**

N-(D-Glucopyranosyl)-N-octadecyl-S-octadecyl-thiocarbamat
Herstellung analog Beispiel 49 aus 8,6 g der Verbindung aus Beispiel 2 und 7,0 g Thiokohlensäurechlorid-S-octadecylester.
$\alpha_D$ = 5,6° (c = 1,0 in Tetrahydrofuran)

**Patentansprüche** für die Vertragsstaaten: BE, CH, DE, FR, GB, IT, LI, NL, SE.

1. Verbindung der allgemeinen Formel I

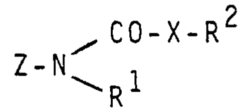

(I)

in der
$R^1$ einen gegebenenfalls substituierten, cyclischen, geradkettigen oder verzweigten gesättigten oder ein- oder mehrfach ungesättigten Kohlenwasserstoffrest mit bis zu 21 C-Atomen darstellt,
$R^2$ einen gegebenenfalls substituierten, aromatischen Kohlenwasserstoffrest oder einen Aralkylrest oder einen gegebenenfalls substituierten, cyclischen, geradkettigen oder verzweigten gesättigten oder ein- oder mehrfach ungesättigten Kohlenwasserstoffrest mit 10 - 25 C-Atomen darstellt,
X für O, S, NH oder NR steht, worin R einen Alkylrest mit bis zu 20 C-Atomen darstellt und
Z einen über das anomere Kohlenstoffatom gebundenen Glycosylrest darstellt.

2. Pharmazeutisch verwendbare Salze der Verbindungen nach Anspruch 1.
3. Verbindungen nach den Ansprüchen 1 oder 2 in denen der Rest $R^1$ 7 - 21 C-Atome hat.
4. Verbindungen nach den Ansprüchen 1 - 3, in denen $R^2$ 10 bis 21 C-Atome hat.
5. Verbindungen nach den Ansprüchen 1 - 4, in denen Z ein Mono- oder Oligosaccharidrest ist.
6. Verfahren zur Herstellung der Verbindungen I gemäß Anspruch 1, dadurch gekennzeichnet, daß man einen Zucker der Formel

Z - OH

wobei die OH-Gruppe an das anomere Kohlenstoffatom des Zuckers gebunden ist, zunächst mit einem Amin der Formel

$R^1$-$NH_2$

worin $R^1$ die in Anspruch 1 angegebene Bedeutung hat zu einem Glycosylamin der Formel II

Z-NH-$R^1$ (II)

am anomeren Kohlenstoffatom umsetzt und dieses Glycosylamin II dann entweder, zur Herstellung der Verbindungen I mit X = O, mit einem Halogenameisensäureester der Formel III

Y-CO-O-$R^2$ (III)

worin Y für Halogen steht und $R^2$ die in Anspruch 1 angegebene Bedeutung hat, umsetzt,

oder, zur Herstellung der Verbindungen I mit X = S, mit einem Thiokohlensäurehalogenid-S-ester der Formel IV

$R^2$-S-CO-Y (IV)

in der Y für Halogen und $R^2$ die oben angegebene Bedeutung hat, umsetzt,

oder, zur Herstellung der Verbindungen I mit X = NH, mit einem Isocyanat der Formel

$R^2$-NCO

worin $R^2$ obengenannte Bedeutung hat, umsetzt,

oder, zur Herstellung der Verbindungen I mit X = NH oder NR, wobei R für Alkyl steht, zunächst mit einem aromatischen Chlorameisensäureester zum Carbamat umsetzt und das Carbamat dann aminolytisch mit einem Amin $R^2$-$NH_2$ oder $R^2$-NH-Alkyl, wobei $R^2$ die obengenannte Bedeutung hat, zum Harnstoff I umsetzt.

7. Arzneimittel enthaltend mindestens eine Verbindung gemäß Anspruch 1.

8. Verwendung der Verbindungen I gemäß Anspruch 1 zur Herstellung eines Arzneimittels zur Behandlung von oder Prophylaxe gegen Krankheiten.

9. Verwendung der Verbindungen I gemäß Anspruch 1 zur Herstellung eines Arzneimittels zur Beeinflussung der körpereigenen Abwehr.

10. Verwendung der Verbindungen I gemäß Anspruch 1 zur Herstellung eines Arzneimittels zur Bekämpfung von Infektionskrankheiten.

**Patentansprüche** für den Vertragsstaat AT.

1. Verfahren zur Herstellung der Verbindungen der allgemeinen Formel

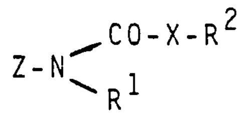

(I)

in der

- $R^1$ einen gegebenenfalls substituierten, cyclischen, geradkettigen oder verzweigten gesättigten oder ein- oder mehrfach ungesättigten Kohlenwasserstoffrest mit bis zu 21 C-Atomen darstellt,
- $R^2$ einen gegebenenfalls substituierten, aromatischen Kohlenwasserstoffrest oder einen Aralkylrest oder einen gegebenenfalls substituierten, cyclischen, geradkettigen oder verzweigten gesättigten oder ein- oder mehrfach ungesättigten Kohlenwasserstoffrest mit 10 - 25 C-Atomen darstellt,
- X für O, S, NH oder NR steht, worin R einen Alkylrest mit bis zu 20, vorzugsweise bis zu 10 C-Atomen darstellt und
- Z einen über das anomere Kohlenstoffatom gebundenen Glycosylrest darstellt,

dadurch gekennzeichnet, daß man einen Zucker der Formel

Z - OH

wobei die OH-Gruppe an das anomere Kohlenstoffatom des Zuckers gebunden ist, zunächst mit einem Amin der Formel

$R^1$-$NH_2$

worin $R^1$ die oben angegebene Bedeutung hat, zu einem Glycosylamin der Formel II

Z-NH-$R^1$ (II)

an anomeren Kohlenstoffatom umsetzt und dieses Glycosylamin II dann entweder, zur Herstellung der Verbindungen I mit X = 0, mit einem Halogenameisensäureester der Formel III

Y-CO-O-$R^2$ (III)

worin Y für Halogen steht und $R^2$ die oben angegebene Bedeutung hat, umsetzt,
oder, zur Herstellung der Verbindungen I mit X = S, mit einem Thiokohlensäurechlorid-S-ester der Formel IV

$R^2$-S-CO-Y (IV)

in der Y für Halogen und $R^2$ die oben angegebene Bedeutung hat, umsetzt,
oder, zur Herstellung der Verbindungen I mit X = NH, mit einem Isocyanat der Formel

$R^2$-NCO

worin $R^2$ obengenannte Bedeutung hat, umsetzt,
oder, zur Herstellung der Verbindungen I mit X = NH oder NR, wobei R für Alkyl steht, zunächst mit einem aromatischen Chlorameisensäureester zum Carbamat umsetzt und das Carbamat dann aminolytisch mit einem Amin $R^2$-$NH_2$ oder $R^2$-NH-Alkyl, wobei $R^2$ die obengenannte Bedeutung hat, zum Harnstoff I umsetzt.

2. Verfahren nach Anspruch 1, dadurch gekennzeichnet, daß der unblockierte Zucker Z-OH, wobei OH für die anomere Hydroxylgruppe steht, mit der in Formel I beschriebenen Bedeutung von Z, in einem geeigneten Lösungsmittel, oder auch ohne Lösungsmittel, gegebenenfalls in Gegenwart eines Katalysators bei Temperaturen zwischen 0°C und 80°C mit ein bis 10 Äquivalenten des betreffenden Amins $R^1$-$NH_2$ umgesetzt wird und das Glycosylamin der Formel II anschließend wie in Anspruch 1 angegeben, weiter umgesetzt wird.

3. Verfahren nach Anspruch 2, dadurch gekennzeichnet, daß das Glycosylamin gemäß Formel II mit 1 bis 5 Äquivalenten eines Halogenameisensäureesters gemäß Formel III umgesetzt wird, wobei man in einen organischen oder wäßrig-organischen Lösungsmittel bei Temperaturen zwischen 0°C und 50°C, gegebenenfalls in Gegenwart einer anorganischen oder organischen Base, arbeitet und nach beendeter Reaktion das Reaktionsprodukt in üblicher Weise isoliert.

4. Verfahren nach Anspruch 1, dadurch gekennzeichnet, daß man das Glycosylamin der Formel II mit 1 bis 10 Äquivalenten des S-substituierten Thiokohlensäurehalogenids der Formel IV in einem organischen Lösungsmittel zur Reaktion gebracht wird, wobei die Reaktionstemperatur zwischen -10°C und 50°C liegt und die Reaktion gegebenenfalls in Gegenwart einer anorganischen oder organischen Base durchgeführt wird.

5. Verfahren nach Anspruch 1, dadurch gekennzeichnet, daß man das Glycosylamin gemäß Formel II mit 1 bis 5 Äquivalenten eines organischen Isocyanats gemäß Formel V zur Reaktion bringt und diese Reaktion in einem inerten organischen Lösungsmittel bei Temperaturen zwischen -20°C und 60°C gegebenenfalls unter Zusatz eines basischen Katalysators, durchführt.

6. Verfahren nach Anspruch 1, dadurch gekennzeichnet, daß man die Reaktion unter Verwendung von Amino-Schutzgruppen durchführt.

7. Verfahren nach Anspruch 6, dadurch gekennzeichnet, daß man Amino-Schutzgruppen vom Typ

$$-\underset{\underset{O}{\|}}{C}-B$$

verwendet, wobei B Trichlormethyl oder Trifluormethyl bedeutet.

8. Verfahren nach Anspruch 6, dadurch gekennzeichnet, daß man als Amino-Schutzgruppen solche vom Typ

$$-\underset{\underset{O}{\|}}{C}-O-E$$

verwendet, wobei E für Ethyl, Trichlorethyl, Benzyl oder tertiär-Butyl steht.

9. Verfahren nach Anspruch 6, dadurch gekennzeichnet, daß man als Amino-Schutzgruppen solche von Sulfenyltyp

-S-G

verwendet, wobei G für Phenyl, substituiertes Phenyl oder Di- oder Triphenylmethyl steht und substituiertes Phenyl einen Phenylrest bezeichnet, der durch einen bis drei Substituenten aus der Reihe Nitro, Niedrigalkyl oder der durch 1 bis 5 Halogenatome substituiert ist.

10. Verfahren nach Anspruch 1, dadurch gekennzeichnet, daß man Zuckerderivate Z-OH als Ausgangsprodukte einsetzt, in welchen die Aminogruppe oder die Aminogruppen zunächst in Form von Acidoresten vorliegen und die Acidoreste durch Protonendonatoren reduziert.

**Claims** for the Contracting States BE, CH, DE, FR, GB, IT, LI, NL, SE

1. Compound of the general formula I

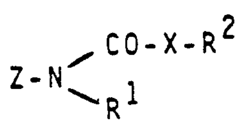

(I)

in which

- $R^1$ represents an optionally substituted, cyclic, straight-chain or branched, saturated or monounsaturated or polyunsaturated hydrocarbon radical having up to 21 C atoms,
- $R^2$ represents an optionally substituted aromatic hydrocarbon radical, an aralkyl radical or an optionally substituted, cyclic, straight-chain or branched, saturated or monounsaturated or polyunsaturated hydrocarbon radical having 10 - 25 C atoms,
- X represents O, S, NH or NR in which R represents an alkyl radical having up to 20 C atoms, and Z represents a glycosyl radical bonded via the anomeric carbon atom.

2. Salts of the compounds according to Claim 1 which can be used for pharmaceutical purposes.

3. Compounds according to Claim 1 or 2 in which the radical $R^1$ has 7 - 21 C atoms.

4. Compounds according to Claims 1 - 3 in which $R^2$ has 10 to 21 C atoms.

5. Compounds according to Claims 1 - 4 in which Z is a monosaccharide or oligosaccharide radical.

6. Process for the preparation of the compounds I according to Claim 1, characterized in that a sugar of the formula

Z-OH

the OH group being bonded to the anomeric carbon atom of the sugar, is first reacted, at the anomeric carbon atom, with an amine of the formula

$R^1$-$NH_2$

in which $R^1$ has the meaning given in Claim 1, to give a glycosylamine of the formula II

Z-NH-$R^1$ (II)

and this glycosylamine II is then either reacted with a halogenoformic acid ester of the formula III

Y-CO-O-$R^2$ (III)

in which Y represents halogen and $R^2$ has the meaning given in Claim 1, in order to prepare the compounds I with X = O, or reacted with a thiocarbonic acid halide S-ester of the formula IV

$R^2$-S-CO-Y (IV)

in which Y represents halogen and $R^2$ has the meaning given above, in order to prepare the compounds I with X = S, or reacted with an isocyanate of the formula

$R^2$-NCO

in which $R^2$ has the abovementioned meaning, in order to prepare the compounds I with X = NH, or, in order to prepare the compounds I with X = NH or NR, R representing alkyl, it is reacted first with an aromatic chloroformic acid ester to give the carbamate and the carbamate is then reacted, in an aminolysis reaction, with an amine $R^2$-$NH_2$ or $R^2$-NH-alkyl, $R^2$ having the abovementioned meaning, to give the urea I.

7. Drugs containing at least one compound according to Claim 1.

8. Use of the compounds I according to Claim 1 for the preparation of a drug for the treatment or prophylaxis of diseases.

9. Use of the compounds I according to Claim 1 for the preparation of a drug for influencing the body's own defences.

10. Use of the compounds I according to Claim 1 for the preparation of a drug for combating infectious diseases.

**Claims** for the Contracting State AT

1. Process for the preparation of the compounds of the general formula I

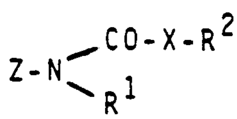

(I)

in which

- $R^1$ represents an optionally substituted, cyclic, straight-chain or branched, saturated or monounsaturated or polyunsaturated hydrocarbon radical having up to 21 C atoms,
- $R^2$ represents an optionally substituted aromatic hydrocarbon radical, an aralkyl radical or an optionally substituted, cyclic, straight-chain or branched, saturated or monounsaturated or polyunsaturated hydrocarbon radical having 10 - 25 C atoms,
- X represents O, S, NH or NR in which R represents an alkyl radical having up to 20 C atoms, preferably up to 10 C atoms, and
- Z represents a glycosyl radical bonded via the anomeric carbon atom,

characterized in that a sugar of the formula

Z-OH

the OH group being bonded to the anomeric carbon atom of the sugar, is first reacted, at the anomeric carbon atom, with an amine of the formula

$R^1$-$NH_2$

in which $R^1$ has the meaning given above, to give a glycosylamine of the formula II

Z-NH-$R^1$ (II)

and this glycosylamine II is then either reacted with a halogenoformic acid ester of the formula III

Y-CO-O-$R^2$ (III)

in which Y represents halogen and $R^2$ has the meaning given above, in order to prepare the compounds I with X = O, or reacted with a thiocarbonic acid halide S-ester of the formula IV

$R^2$-S-CO-Y (IV)

in which Y represents halogen and $R^2$ has the meaning given above, in order to prepare the compounds I with X = S, or reacted with an isocyanate of the formula

$R^2$-NCO

in which R has the abovementioned meaning, in order to prepare the compounds I with X = NH, or, in order to prepare the compounds I with X = NH or NR, R representing alkyl, it is first reacted with an aromatic chloroformic acid ester to give the carbamate and the carbamate is then reacted, in an aminolysis reaction, with an amine $R^2$-$NH_2$ or $R^2$-NH-alkyl, $R^2$ having the abovementioned meaning, to give the urea I.

2. Process according to Claim 1, characterized in that the unblocked sugar Z-OH, OH representing the anomeric hydroxyl group and Z having the meaning described in the formula I, is reacted with one to 10 equivalents of the particular amine $R^1$-$NH_2$, in a suitable solvent or without a solvent, if appropriate in the presence of a catalyst, at temperatures of between 0°C and 80°C, and the glycosylamine of the formula II is then reacted further as indicated in Claim 1.

3. Process according to Claim 2, characterized in that the glycosylamine according to the formula II is reacted with 1 to 5 equivalents of a halogenoformic acid ester according to the formula III, the reactiön being carried out in an organic or aqueous-organic solvent, at temperatures of between 0°C and 50 C, if appropriate in the presence of an inorganic or organic base, and the reaction product is isolated in the usual manner after the reaction has ended.

4. Process according to Claim 1, characterized in that the glycosylamine of the formula II is reacted with 1 to 10 equivalents of the S-substituted thiocarbonic acid halide of the formula IV, in an organic solvent, the reaction temperature being between -10°C and 50°C and the reaction being carried out, if appropriate, in the presence of an inorganic or organic base.

5. Process according to Claim 1, characterized in that the glycosylamine according to the formula II is reacted with 1 to 5 equivalents of an organic isocyanate according to the formula V, and this reaction is carried out in an inert organic solvent, at temperatures of between -20°C and 60°C, if appropriate with the addition of a basic catalyst.

6. Process according to Claim 1, characterized in that the reaction is carried out using amino-protecting groups.

7. Process according to Claim 6, characterized in that amino-protecting groups of the type

$$-\underset{\underset{O}{\|}}{C}-B$$

are used, B denoting trichloromethyl or trifluoromethyl.

8. Process according to Claim 6, characterized in that the amino-protecting groups used are of the type

$$-\underset{\underset{O}{\|}}{C}-O-E$$

E representing ethyl, trichloroethyl, benzyl or tertiary butyl.

9. Process according to Claim 6, characterized in that the amino-protecting groups used are of the sulphenyl type

-S-G

G representing phenyl, substituted phenyl, diphenylmethyl or triphenylmethyl, and substituted phenyl denoting a phenyl radical which is substituted by one to three substituents from the group comprising nitro and lower alkyl or by 1 to 5 halogen atoms.

10. Process according to Claim 1, characterized in that the starting materials used are sugar derivatives Z-OH in which the amino group or the amino groups are initially in the form of azido radicals, and the azido radicals are reduced by proton donors.

**Revendications** pour les Etats contractants: BE, CH, DE, FR, GB, IT, LI, NL, SE

1. Composé de formule générale (I)

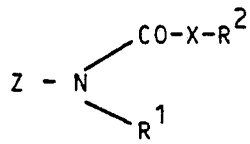 (I)

dans laquelle

$R^1$ représente un reste d'hydrocarbure comportant jusqu'à 21 atomes de carbone, cyclique, linéaire ou ramifié, éventuellement substitué, saturé ou présentant un ou plusieurs insaturations,

$R^2$ représente un reste d'hydrocarbure aromatique éventuellement substitué ou un reste aralkyle ou un reste d'hydrocarbure cyclique, linéaire ou ramifié, éventuellement substitué, saturé ou insaturé une ou plusieurs fois et comportant 10 à 25 atomes de carbone,

X représente O, S, NH ou NR, R étant un reste alkyle ayant jusqu'à 20, avantageusement jusqu'à 10 atomes de carbone, et

Z représente un reste glycosyle relié par l'intermédiaire de l'atome de carbone anomère.

2. Sels, pharmaceutiquement utilisables, des composés selon la revendication 1.
3. Composés selon les revendications 1 ou 2, dans lesquels le reste $R^1$ comporte 7 à 21 atomes de carbone.
4. Composés selon les revendications 1 à 3, dans lesquels $R^2$ comporte 10 à 21 atomes de carbone.
5. Composés selon les revendications 1 à 4, dans lesquels Z représente un reste monosaccharide ou oligosaccharide.
6. Procédé pour la préparation des composés (I) selon la revendication 1, caractérisé en ce qu'on fait réagir sur l'atome de carbone anomère un sucre de formule

Z - OH

où
le groupe OH est fixé sur l'atome de carbone anomère du sucre, tout d'abord avec une amine de formule

$R^1$ -$NH_2$

dans laquelle
$R^1$ a le sens indiqué à la revendication 1, pour obtenir une glycosylamine de formule (II)

Z-NH-$R^1$ (II)

et l'on fait ensuite réagir cette glycosylamine (II), pour préparer les composés (I) dans lesquels X représente O, avec un ester d'acide halogénoformique de formule (III)

Y-CO-O-$R^2$ (III)

dans laquelle
Y représente un atome d'halogene et $R^2$ a le sens indiqué à la revendication 1,
ou bien, pour préparer les composés (I) dans lesquels X représente S, on fait réagir cette glycosylamine (II) avec un S-ester d'halogénure d'acide thiocarbonique de formule (IV)

$R^2$-S-CO-Y (IV)

dans laquelle
Y représente un halogène et $R^2$ a le sens précité,
ou bien, pour préparer les composés (I) dans lesquels X représente NH, on fait réagir la glycosylamine (II) avec un isocyanate de formule

$R^2$-NCO

dans laquelle
$R^2$ a le sens précité,
ou bien, pour préparer les composés (I) dans lesquels X représente NH ou NR, R représentant un groupe alkyle, on fait réagir la glycosylamine (II) tout d'abord avec un ester aromatique d'acide chloroformique pour obtenir un carbamate et l'on fait ensuite réagir ce carbamate, avec aminolyse, avec une amine R-$NH_2$ ou $R^2$-NH-alkyle, où $R^2$ a le sens précité, pour obtenir l'urée (I).
7. Médicament contenant au moins un composé selon la revendication 1.
8. Utilisation des composés (I) selon la revendication 1 pour préparer un médicament destiné à un traitement curatif ou prophylactique de maladies.

9. Utilisation des composés (I) selon la revendication 1 pour préparer un médicament destiné à influencer la défense endogène de l'organisme.

10. Utilisation des composés (I) selon la revendication 1 pour la préparation d'un médicament destiné à la lutte contre les maladies infectieuses.

**Revendications** pour Etat contractant: AT

1. Procédé de préparation des composés de formule générale (I)

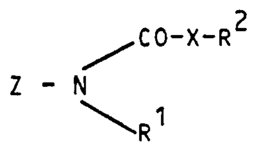

(I)

dans laquelle

$R^1$ représente un reste d'hydrocarbure comportant jusqu'à 21 atomes de carbone, cyclique, linéaire ou ramifié, éventuellement substitué, saturé ou présentant un ou plusieurs insaturations,

$R^2$ représente un reste d'hydrocarbure aromatique éventuellement substitué ou un reste aralkyle ou un reste d'hydrocarbure cyclique, linéaire ou ramifié, éventuellement substitué, saturé ou insaturé une ou plusieurs fois et comportant 10 à 25 atomes de carbone,

X représente O, S, NH ou NR, R étant un reste alkyle ayant jusqu'à 20, avantageusement jusqu'à 10 atomes de carbone, et

Z représente un reste glycosyle relié par l'intermédiaire de l'atome de carbone anomère,

procédé caractérisé en ce qu'on fait réagir sur l'atome de carbone anomère un sucre de formule

Z - OH

où

le groupe OH est fixé sur l'atome de carbone anomère du sucre, tout d'abord avec une amine de formule

$R^1$-$NH_2$

dans laquelle

$R^1$ a le sens précité, pour obtenir une glycosylamine de formule (II)

Z-NH-$R^1$ (II)

et l'on fait ensuite réagir cette glycosylamine (II), pour préparer les composés (I) dans lesquels X représente O, avec un ester d'acide halogénoformique de formule (III)

Y-CO-O-$R^2$ (III)

dans laquelle

Y représente un atome d'halogène et $R^2$ a le sens précité,

ou bien, pour préparer les composés (I) dans lesquels X représente S, on fait réagir la glycosylamine (II) avec un S-ester de chlorure d'acide thiocarbonique de formule (IV)

$R^2$-S-CO-Y (IV)

dans laquelle

Y représente un halogène et $R^2$ a le sens précité,

ou bien, pour préparer les composés (I) dans lesquels X représente NH, on fait réagir la glycosylamine avec un isocyanate de formule

$R^2$-NCO

dans laquelle

$R^2$ a le sens précité,

ou bien, pour préparer les composés (I) dans lesquels X représente NH ou NR, R représentant un groupe alkyle, on fait réagir la glycosylamine tout d'abord avec un ester aromatique d'acide chloroformique pour obtenir un carbamate et l'on fait ensuite réagir ce carbamate, avec aminolyse, avec une amine $R^2$-$NH_2$ ou $R^2$-NH-alkyle, ou $R^2$ a le sens précité, pour obtenir l'urée (I).

2. Procédé selon la revendication 1, caractérisé en ce qu'on fait réagir le sucre Z-OH non bloqué ou non coiffé, dans lequel OH représente le groupe hydroxyle anomère, Z ayant le sens décrit à propos de la formule (I), dans un solvant convenable, ou également sans solvant, éventuellement en présence d'un catalyseur à des températures comprises entre 0° C et 80° C, avec 1 à 10 équivalents de l'amine $R^1$-$NH_2$ correspondante et l'on fait encore réagir la glycosylamine de formule (II), ensuite, comme indiqué à la revendication 1.

3. Procédé selon la revendication 2, caractérisé en ce qu'on fait réagir la glycosylamine répondant à la formule (II) avec 1 à 5 équivalents d'un ester d'acide halogénoformique répondant à la formule (III), en opérant dans un solvant organique ou hydroorganique à des températures comprises entre 0° C et 50° C, éventuellement en présence d'une base minérale ou organique, et, après achèvement de la réaction, on isole de façon usuelle le produit de la réaction.

4. Procédé selon la revendication 1, caractérisé en ce qu'on fait réagir dans un solvant organique la glycosylamine de formule (II) avec 1 à 10 équivalents de l'halogénure d'acide thiocarbonique substitué sur le soufre, de formule (IV), la température de réaction se situant entre -10° C et +50° C et la réaction étant conduite éventuellement en présence d'une base minérale ou organique.

5. Procédé selon la revendication 1, caractérisé en ce qu'on fait réagir la glycosylamine de formule (II) avec 1 à 5 équivalents d'un isocyanate organique de formule (V), et l'on conduit cette réaction dans un solvant organique inerte, à des températures comprises entre -20° C et +60° C, éventuellement avec addition d'un catalyseur basique.

6. Procédé selon la revendication 1, caractérisé en ce qu'on conduit la réaction en utilisant des groupes protecteurs de la fonction amine.

7. Procédé selon la revendication 6, caractérisé en ce qu'on utilise des groupes protecteurs de fonction amino de type

$$-\underset{\underset{O}{\|}}{C}-B$$

où
B représente un groupe trichlorométhyle ou trifluorométhyle.

8. Procédé selon la revendication 6, caractérisé en ce qu'on utilise, comme groupes protecteurs de fonction amine, ceux du type

$$-\underset{\underset{O}{\|}}{C}-O-E$$

où
E représente un groupe éthyle, trichloréthyle, benzyle ou tertiobutyle.

9. Procédé selon la revendication 6, caractérisé en ce qu'on utilise, comme groupes protecteurs de fonction amino, ceux du type sulfényle

-S-G

où
G représente un groupe phényle, phényle substitué ou diphénylméthyle ou triphénylméthyle et l'expression de groupe phényle substitué désigne un reste phényle qui porte 1 à 3 substituants choisis dans la série des groupes nitro, alkyle inférieur ou qui porte 1 à 5 atomes d'halogène(s) comme substituant(s).

10. Procédé selon la revendication 1, caractérisé en ce qu'on utilise, comme produit de départ, des dérivés de sucre Z-OH dans lesquels le ou les groupes amino sont tout d'abord présents sous forme de restes acides, et l'on réduit les restes acides à l'aide de donneurs de protons.